# EUROPÄISCHE PATENTSCHRIFT

(11) **EP 1 230 649 B1**
(45) Veröffentlichungstag und Bekanntmachung des Hinweises auf die Patenterteilung: **11.06.2003**
(21) Anmeldenummer: 00975983.8
(22) Anmeldetag: 02.11.2000
(51) Int. Cl.: H01F 5/00

(54) **SPULE**
COIL
BOBINE

(30) Priorität: 11.11.1999 DE 19954367; 10.02.2000 DE 10005917
(43) Veröffentlichungstag der Anmeldung: 14.08.2002
(73) Patentinhaber: Reichwein, Dietrich, 5700 Zell am See (AT); Peters, Olaf, 9772 Dellach/an der Drau (AT)
(72) Erfinder: PETERS, Olaf, A-9772 Dellach (AT)
(74) Vertreter: Pfenning, Meinig & Partner GbR
(86) Internationale Anmeldenummer: EP0010781
(87) Internationale Veröffentlichungsnummer: WO01035425

(56) Entgegenhaltungen:
- DE-A- 2 405 383
- DE-A- 19 543 573
- DE-U- 29 608 779
- GB-A- 1 275 859
- PATENT ABSTRACTS OF JAPAN vol. 014, no. 263 (E-0938), 7. Juni 1990 (1990-06-07) & JP 02 081404 A (ISAO OSAKABE), 22. März 1990 (1990-03-22)

## Beschreibung

Die vorliegende Erfindung bezieht sich auf eine Spule, wie sie zur Erzeugung von skalaren Feldern eingesetzt werden kann.

Aus dem Stand der Technik ist die Klein'sche Wicklung oder auch Möbius-Spule (August Ferdinand Möbius, Deutscher Mathematiker und Astronom, 17.11.1790 - 26.9.1868) bekannt (Sinichi SEIKE in "The Principles of Ultrarelativity", Space Research Institute, Ninomiya Press 1994).

Entwickelt wurde diese Spulenform, weil das Magnetfeld dieser Wicklung unter Gleichstrom gesetzt ein Feld erzeugt, das der Topologie einer Klein'schen Flasche (Felix Klein, Deutscher Mathematiker 25.4.1849 - 22.6.1925) entspricht. Dabei bildet eine Spule, die zur Hälfte links und zur anderen Hälfte rechts gewickelt ist, einen magnetischen Quasi-Single-Pol mit einer Feldstärkenaufteilung, bei der zwei gleiche Pole am Ende und der Gegenpol in der Mittel der Spule lokalisiert sind. Dabei bilden jeweils ¹/₆ des

Endfeldes mit dem ¹/₃ des Mittelpoles geschlossene Feldlinien. Jeweils ¹/₆des Feldes am Ende der Spule besitzt eine Divergenz unendlich (div ∞) und benimmt sich daher wie eine elektrische Feldlinie. Dieses Verhalten führt zu unterschiedlichsten Phänomen, die für Raumphysik und Biologie gleichermaßen von Bedeutung sind.

Diese Wickelform entsteht, wenn die einzelnen Windungen in Form von "halben Schlägen" um den Spulenkern gelegt werden.

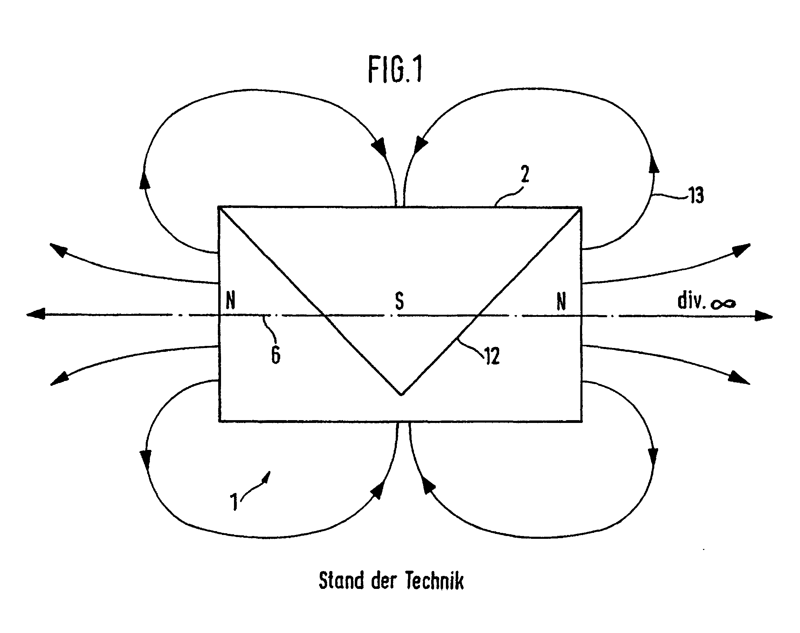

Eine derartige Möbius-Spule ist in Figur 1 dargestellt, wobei eine Spule 1 einen Spulenkörper 2 aufweist, um den ringförmig in Art einer herkömmlichen Spule einzelne Windungen eines elektrischen Leiters gelegt sind. Allerdings werden diese einzelnen Windungen im Unterschied zum Stand der Technik in Form von "halben Schlägen" um den Spulenkörper 2 gelegt, so daß sich eine V-förmige Knotenlinie 12 ausbildet.

Diese Wicklungsart gestattet es jedoch nicht, eine bifilare Wicklung zu erstellen.

Aufgabe der vorliegenden Erfindung ist es daher, eine Spule zur Verfügung zu stellen, mit der Skalarfelder erzeugt werden können.

Diese Aufgabe wird durch die Spule nach Anspruch 1 gelöst. Vorteilhafte Weiterbildungen der erfindungsgemäßen Spule werden in den abhängigen Ansprüchen gegeben.

Die erfindungsgemäße, vorteilhafterweise zylindrische Spule (Mehrfachkleinspule) besitzt Wicklungen einer ersten elektrischen Leitung und einer weiteren, beispielsweise zweiten elektrischen Leitung, wobei die Leitungen an ihrem Ende in funktionsgerechter Weise miteinander verschaltet, im Falle einer bifilaren Klein'schen Spule mit einer ersten und einer zweiten Leitung die Leitungen an einem Ende der Spule elektrisch miteinander verbunden sind, so daß in letzterem Falle die eine Leitung als Hinleiter und die zweite Leitung als Rückleiter dienen kann. Die Spule ist dabei so gewickelt, daß die einzelnen Wicklungen der einzelnen elektrischen Leitungen längs des Umfangs des Spulenkörpers gegeneinander versetzt beginnen. Dies kann im Fall von zwei Leitungen vorteilhafterweise mit einem Versatz von 180 ° erfolgen, so daß die einzelnen Wicklungen der ersten elektrischen Leitung gegenüber den Wicklungen der zweiten elektrischen Leitung auf dem Spulenkörper gegenüberliegend beginnen.

Dabei wird bei jeder der einzelnen Leitungen nach etwa einem Wicklungsumlauf eine Umlenkstelle gebildet, indem die Leitung nach einem Umlauf unter sich selbst durchgeführt, anschließend über die in Spulenachsenrichtung benachbarten anderen Leitungen geführt und dann parallel zu diesen anderen Leitungen weiter um den Spulenkörper gewickelt wird. Dadurch folgen die Wicklungen der ersten elektrischen Leitung und der weiteren elektrischen Leitung jeweils in Spulenachsenrichtung abwechselnd aufeinander. Die so gebildeten Umlenkstellen (Knoten) können längs der Achse der Spule linear oder auch zickzagförmig, beispielsweise in V-Form, angeordnet sein. Vorteilhafterweise ist dabei die Knotenlinie jeweils V-förmig angeordnet, wobei an der Spitze des V's ein Richtungswechsel der elektrischen Leitungen erfolgt, so daß beispielsweise bisher rechtsläufige Wicklungen in linksläufige Wicklungen überführt werden.

Mit anderen Worten wird bei der erfindungsgemäßen Spule mit zwei Drähten, von diametraler Position ausgehend, gleichsinning-wechselweise je ein halber Schlag gelegt. Am Spulenende werden die Drahtenden miteinander verbunden, so daß in zwei benachbarten Windungen entgegengesetzte Stromrichtung bei angelegter Spannung vorgegeben ist. Somit heben sich die magnetischen Felder gegenseitig auf. In Vektordarstellung entfällt das Argument des Magnetfeldvektors, d.h. es wird exakt 0, da dem zweiten Kirchhoff'schen Gesetz zufolge Strom und Gegenstrom idente Größenordnungen aufweisen.

Bei Feldern, bei denen die Argumente der Feldvektoren gleich Null sind, wird von Skalarfeldern gesprochen. Diese sind im Falle der erfindungsgemäßen Spule zwangsläufig deshalb präsent, da aufgrund des Energieerhaltungssatzes die eingesetzte elektrische Energie nicht verschwinden kann (K. Meyl: "Elektromagnetische Umweltverträglichkeit, Ursachen, Phänomene und naturwissenschaftliche Konsequenzen. Umdruck zur Vorlesung", ISBN 3-9802-642-8-3 und ISBN 3-9802-542-9-1, sowie K. Meyl "Potentialwirbel" Band 1 und 2, ISBN 3-9802-542-1-6 und ISBN 3-9802-542-2-4).

Im folgenden werden einige Beispiele der erfindungsgemäßen Spulen beschrieben werden.

Es zeigen
Figur 1 eine herkömmliche Möbiusspule;
Figur 2 eine erfindungsgemäße Spule;
   und
Figur 3 zwei Wicklungsarten der erfindungsgemäßen Spule.

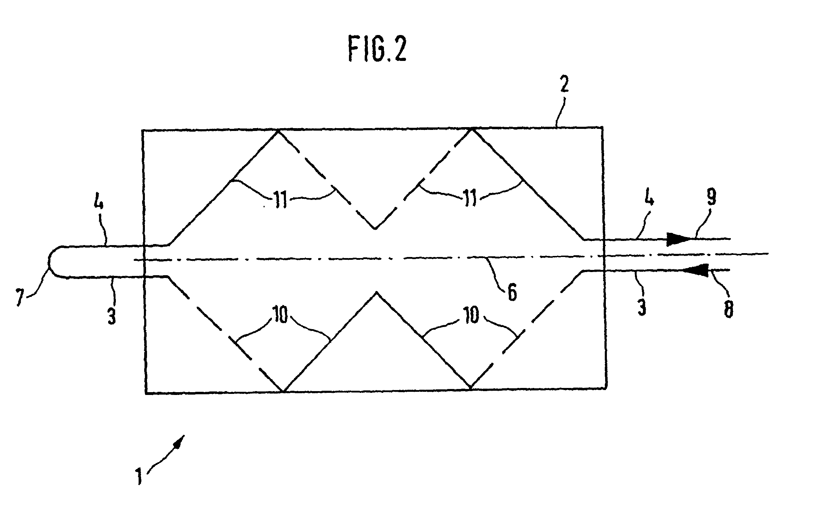

Figur 2 zeigt eine Spule 1 mit einem Spulenkörper 2, auf den zwei elektrische Leiter 3 und 4 in Form einer Spule aufgewickelt sind. Dabei werden die Leiter 3 und 4, wie oben beschrieben, gewickelt, so daß jeweils eine Leitung 3 neben einer Leitung 4 auf einem Umlauf zu liegen kommen. Die Knoten der Leitung 3 ordnen sich in Form der V-förmigen Knotenlinie 10 an, wobei hier in der Darstellung zu beachten ist, daß die durchgezogenen Linien die unmittelbare Aufsicht des Betrachters darstellen, während die gestrichelten Linien der Knotenlinie 10 sich auf der Rückseite des Spulenkörpers 2 fortsetzen. In gleicher Weise ergibt sich die um 180° längs des Umfangs des Spulenkörpers versetzte Knotenlinie 11 der Leitung 4. Die Leitungen 3 und 4 weisen nunmehr jeweils einen Anschluß 8 bzw. 9 auf und sind am anderen Ende der Spule an einer Umkehrschlaufe 7 elektrisch miteinander verbunden.

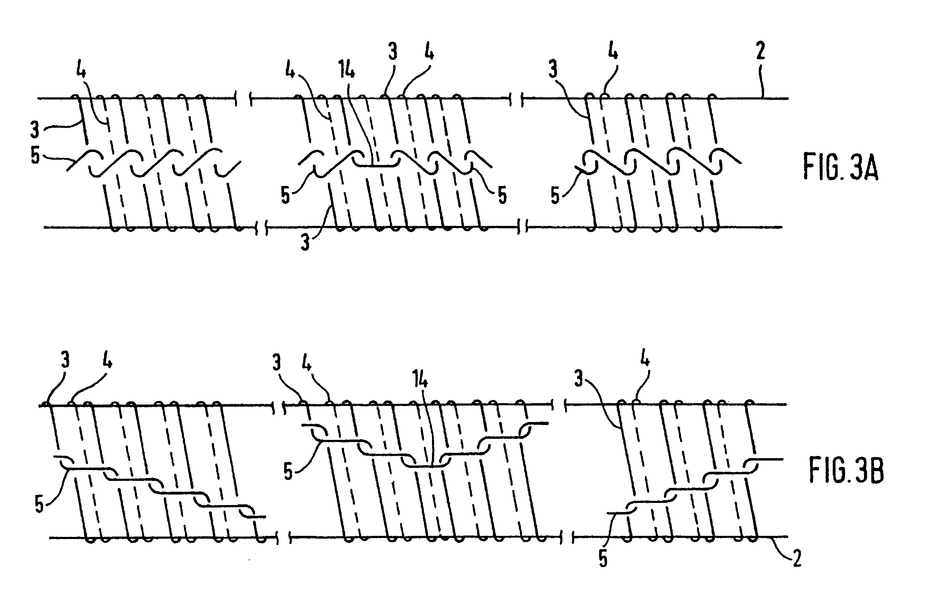

Figur 3 zeigt in Teilbild A und Teilbild B jeweils die Knotenbildung gemäß der Erfindung. In Figur 3A ist eine Knotenlinie gezeigt, die sich in axialer Richtung des Spulenkörpers 2 linear erstreckt.

Die Leitung 3 wird dabei einmal mehr um den Spulenkörper 2 gewickelt und dann unter sich selbst durchgezogen und über diese Leitung und auch über die benachbarte zweite Leitung 4 hinweggeführt, worauf sie dann in einer neuer Windung um den Spulenkörper 2 geführt wird. Dasselbe erfolgt in symmetrischer Weise mit der Leitung 4. Dabei ergeben sich die Knoten (Umlenkstellen) 5. Die Umlenkstellen 5 werden nunmehr nebeneinander plaziert, so daß sie in einer Linie in axialer Richtung des Spulenkörpers 2 zu liegen kommen. In der Mitte der Figur 3A ist dargestellt, wie die Leitung 3 geführt wird, so daß sie eine Richtungswechselstelle bildet. Das heißt, die Leitung 3, die bisher rechtsdrehend gewickelt war, ist anschließend an die Umkehrstelle linksdrehend gewickelt. Die Knoten 5, die sich hieran anschließen an diese Umlenkstelle 14, sind in der beschriebenen Weise hergestellt.

Die zweite Leitung 4 ist in Figur 3 lediglich gestrichelt eingezeichnet. Für sie ergibt sich auf der Rückseite des Spulenkörpers 2 eine entsprechende Knotenlinie, die hier jedoch nicht dargestellt ist.

Wird die Knotenlinie linear geführt, so erzeugt die Spule bei Anlegen eines elektrischen Stromes an die Spule einen magnetischen Dipol.

In Figur 3 ist dargestellt, wie die Knoten auch in V-Form geführt werden können. Dabei ist jeder einzelne Knoten in Umfangsrichtung des Spulenkörpers 2 gegenüber dem benachbarten Knoten um eine geringe Distanz versetzt. In der Mitte von Figur 3B ist dargestellt, wie durch die Erzeugung einer Richtungswechselstelle 14 die typische V-Form entsteht. Die Richtungswechselstelle 14 ist dabei die Spitze des V's.

Wird die erfindungsgemäße Spule wie in Figur 3B so ausgeführt, daß sich eine V-Form der Umlenkstellen 5 ergibt, so erzeugt die Spule, wenn an sie ein elektrischer Strom angelegt wird, einen magnetischen Tripol.

## Patentansprüche

1. Spule(1), mit Wicklungen einer ersten elektrischen Leitung(3) und Wicklungen mindestens einer weiteren elektrischen Leitung(4), wobei die elektrischen Leitungen(3, 4) an ihren Einzelenden in funktionsgerechter Weise miteinander verschaltet sind, **dadurch gekennzeichnet, daß** die einzelnen Wicklungen der ersten elektrischen Leitung(3) und der mindestens einen weiteren elektrischen Leitung(4) längs des Umfangs des Spulenkörpers(2) gegeneinander versetzt beginnen, und jede der Leitungen(3, 4) nach etwa einem Wicklungsumlauf eine Umlenkstelle (5) bildend unter sich selbst durchgeführt, über die in Spulenlängsrichtung benachbarten anderen Leitungen geführt und parallel zu den anderen Leitungen um den Spulenkörper(2) gewickelt ist derart, daß in axialer Richtung des Spulenkörpers(2) die Wicklungen verschiedener Leitungen jeweils in vorbestimmter Abfolge abwechselnd aufeinanderfolgen.

2. Spule(1) nach dem vorhergehenden Anspruch, **dadurch gekennzeichnet, daß** eine erste elektrische Leitung(3) und eine zweite elektrische Leitung(4) als weitere elektrische Leitung um den Spulenkörper(2) gewickelt sind, wobei die beiden elektrischen Leitungen(3, 4) an einem Ende der Spule(1) elektrisch miteinander verbunden(7) sind.

3. Spule(1) nach einem der vorhergehenden Ansprüche, **dadurch gekennzeichnet, daß** in axialer Richtung der Spule(1) mindestens einmal die Richtung der Wicklung mindestens einer der elektrischen Leitungen(3, 4) umgekehrt wird.

4. Spule(1) nach dem vorhergehenden Anspruch, **dadurch gekennzeichnet, daß** die Richtung der Wicklung an einer Umlenkstelle(5) umgekehrt wird.

5. Spule(1) nach einem der vorhergehenden Ansprüche, **dadurch gekennzeichnet, daß** die Umlenkstellen(5) der ersten elektrischen Leitung(3) gegenüber den Umlenkstellen(5) der weiteren elektrischen Leitung(4) längs des Umfangs der Spule(1) um ca. 180 ° versetzt sind.

6. Spule(1) nach einem der vorhergehenden Ansprüche, **dadurch gekennzeichnet, daß** die Umlenkstellen(5) der ersten Leitung(3) und/oder der weiteren Leitung(4) eine gerade Linie in axialer Richtung der Spule(1) bilden.

7. Spule(1) nach einem der vorhergehenden Ansprüche, **dadurch gekennzeichnet, daß** die Umlenkstellen(5) der ersten und/oder der weiteren Leitung(3, 4) in axialer Richtung zickzack-förmig angeordnet sind.

8. Spule (1) nach dem vorhergehenden Anspruch, **dadurch gekennzeichnet, daß** die Umlenkstellen(5) der ersten und/oder der weiteren Leitung(3, 4) in axialer Richtung V-förmig angeordnet sind.

9. Spule(1) nach einem der beiden vorhergehenden Ansprüche, **dadurch gekennzeichnet, daß** an den Stellen, an denen die Umlenkstellen(5) unter einem Winkel aufeinanderstoßen, die Richtung der Wicklung der jeweiligen umgelenkten Leitung(3, 4) umgekehrt wird.

10. Spule(1) nach einem der vorhergehenden Ansprüche, **dadurch gekennzeichnet, daß** die Spule(1) zylindrisch ist.

## Claims

1. Coil (1), having windings of a first electrical conductor (3) and windings of at least one further electrical conductor (4), with the electrical conductors (3, 4) being connected to one another at their individual ends in a manner appropriate to their function, **characterized in that** the individual windings of the first electrical conductor (3) and of the at least one further electrical conductor (4) start offset with respect to one another along the circumference of the coil former (2), and each of the conductors (3, 4) is passed through under itself, forming a reversal point (5), after approximately one winding cycle, is passed over the other conductors which are adjacent in the coil longitudinal direction and is wound around the coil former (2) parallel to the other conductors in such a way that the windings of different conductors each follow one another alternately in a predetermined sequence in the axial direction of the coil former (2).

2. Coil (1) according to the preceding claim, **characterized in that** a first electrical conductor (3) and a second electrical conductor (4), as further electrical conductor, are wound around the coil former (2), with the two electrical conductors (3, 4) being electrically connected (7) to one another at one end of the coil (1).

3. Coil (1) according to one of the preceding claims, **characterized in that** the direction of the winding of at least one of the electrical conductors (3, 4) is reversed at least once in the axial direction of the coil (1).

4. Coil (1) according to the preceding claim, **characterized in that** the direction of the winding is reversed at a reversal point (5).

5. Coil (1) according to one of the preceding claims, **characterized in that** the reversal points (5) of the first electrical conductor (3) are offset through about 180° with respect to the reversal points (5) of the further electrical conductor (4) along the circumference of the coil (1).

6. Coil (1) according to one of the preceding claims, **characterized in that** the reversal points (5) of the first conductor (3) and/or of the further conductor (4) form a straight line in the axial direction of the coil (1).

7. Coil (1) according to one of the preceding claims, **characterized in that** the reversal points (5) of the first and/or of the further conductor (3, 4) are arranged in a zigzag shape in the axial direction.

8. Coil (1) according to the preceding claim, **characterized in that** the reversal points (5) of the first and/or the further conductor (3, 4) are arranged in a V-shape in the axial direction.

9. Coil (1) according to one of the two preceding claims, **characterized in that** the direction of the winding of the respective reversed conductor (3, 4) is reversed at the points at which the reversal points (5) abut against one another at an angle.

10. Coil (1) according to one of the preceding claims, **characterized in that** the coil (1) is cylindrical.

## Revendications

1. Bobine (1) ayant des enroulements d'un premier conducteur électrique (3) et des enroulements d'au moins un autre conducteur électrique (4), où les extrémités individuelles des conducteurs électriques (3, 4) sont branchées l'une à l'autre selon leur fonction, **caractérisée en ce que** les différents enroulements du premier conducteur électrique (3) et dudit au moins un autre conducteur électrique (4) commencent de façon décalée les uns par rapport aux autres, le long de la circonférence du corps de bobine (2), et chacun des conducteurs (3, 4) passe sous lui-même en formant un point de renvoi (5) au bout d'environ un tour d'enroulement, par-dessus les autres conducteurs contigus, dans le sens longitudinal de la bobine, et est enroulé parallèlement auxdits autres conducteurs, autour du corps de bobine (2), de manière telle que les enroulements des différents conducteurs se succèdent dans le sens axial du corps de bobine (2) selon une séquence prédéterminée.

2. Bobine (1) selon la revendication précédente, **caractérisée en ce qu'**un premier conducteur électrique (3) et un deuxième conducteur électrique (4) constituant l'autre conducteur électrique sont enroulés autour du corps de bobine (2), les deux conducteurs électriques (3, 4) étant électriquement reliés l'un à l'autre (7) à une extrémité de la bobine (1).

3. Bobine (1) selon l'une des revendications précédentes, **caractérisée en ce que**, dans le sens axial de la bobine (1), la direction d'enroulement d'au moins un des conducteurs électriques (3, 4) est inversée au moins une fois.

4. Bobine (1) selon la revendication précédente, **caractérisée en ce que** la direction d'enroulement est inversée dans un point de renvoi (5).

5. Bobine (1) selon l'une des revendications précédentes, **caractérisée en ce que** les points de renvoi (5) du premier conducteur électrique (3) sont décalés d'environ 180°, dans le sens de la circonférence de la bobine (1), par rapport aux points de renvoi (5) de l'autre conducteur électrique (4).

6. Bobine (1) selon l'une des revendications précédentes, **caractérisée en ce que** les points de renvoi (5) du premier conducteur (3) et/ou de l'autre conducteur (4) forment une ligne droite dans le sens axial de la bobine (1).

7. Bobine (1) selon l'une des revendications précédentes, **caractérisée en ce que** les points de renvoi (5) du premier conducteur et/ou de l'autre conducteur (3, 4) sont disposés selon une ligne en zigzag dans le sens axial.

8. Bobine (1) selon la revendication précédente, **caractérisée en ce que** les points de renvoi (5) du premier et/ou de l'autre conducteur (3, 4) sont disposés selon une ligne en V.

9. Bobine (1) selon l'une des deux revendications précédentes, **caractérisée en ce que** le sens d'enroulement du conducteur retourné (3, 4) est inversé aux endroits où les points de renvoi correspondants (5) se touchent en formant un angle.

10. Bobine (1) selon l'une des revendications précédentes, **caractérisée en ce que** la bobine (1) est cylindrique.
